# EUROPEAN PATENT SPECIFICATION

(11) **EP 0 408 690 B1**
(45) Date of publication and mention of the grant of the patent: **10.02.1999**
(21) Application number: 90900043.2
(22) Date of filing: 12.12.1989
(51) Int. Cl.: C12N 15/04, C12N 1/18, C12G 1/022

(54) **L-MALIC ACID DEGRADING YEAST FOR WINE MAKING**
L-APFELSÄURE ABBAUENDE HEFE ZUR WEINHERSTELLUNG
LEVURE DEGRADANT L'ACIDE L-MALIQUE POUR LA PRODUCTION DU VIN

(30) Priority: 27.01.1989 BR 8900480
(43) Date of publication of application: 23.01.1991
(73) Proprietor: UNIVERSIDADE DE CAXIAS DO SUL, 95070-Caxias de Sul, RS (BR)
(72) Inventor: CARRAU, Juan, L., 95070-Caxias do Sul, RS (BR); DILLON, Aldo, José, Pinheiro, 95070-Caxias do Sul, RS (BR); SERAFINI, Luciana, Atti, 95100-Caxias do Sul, RS (BR); PASQUAL, Mirian, Salvador, 95100-Caxias do Sul, RS (BR)
(74) Representative: Moinas, Michel
(86) International application number: PCT/BR89/00013
(87) International publication number: WO 90/08820

(56) References cited:
- Applied and Environmental Microbiology, Vol. 51, No. 5, 1986 Alfonso Pina et al: "Intergeneric Hybrids of Saccharomyces cerevisiae and Zygosaccharomyces fermentati Obtained by Protoplast Fusion ", see page 995 - page 1003
- Reviska Microbiol. de ogia, Vol. 12, No. 3, 1981 Juan L. Carrau et al: "El periodo de latencia de Schizosaccharomyces pombe ", see page 105 - page 109
- Chemical Abstracts, volume 110, no. 8, 20 February 1989, (Columbus, Ohio, US), Lee, Jong Soo et al : "Characterization of fusant from protoplast fusion between Saccharomyces cerevisiae D-71 and Zygosaccharomyces rouxii SR-S ", see page 597, abstract 6333x, & Sanop Misaengmul Hakhoechi 1988, 16( 4), 297- 302V
- Chemical Abstracts, volume 98, no. 5, 31 January 1983, (Columbus, Ohio, US), Vezinhet, Francoise : "Action of some medium factors on malic acid metabolism by Saccharomyces and Schizosaccharomyces during alcoholic fermentation ", see page 531, abstract 32970f, & Sci.Aliments 1982, 2( 3), 297- 312V
- Derwent's abstract, No. 85-120 728/20, SU 1 045 630, publ. week 8520, & SU-A-1 045 630

## Description

### Descriptive Report of the Invention Patent " L-MALIC ACID DEGRADING YEAST FOR WINE MAKING":

This invention deals with a new yeast obtained by fusion between Sacoharomyces cerevisiae and Schizosaccharomyces pombe (MB7TCα), which has the ability to degrade malic acid, and of another new yeast obtained by retrofusion between the above fusion product and the parental strain Saccharomyces cerevisiae, as well as new yeasts obtained by fusion between product MB7TC and a yeast with the Killer phenotype and another pectinase-producing Saccharomyces cerevisiae yeast, all of them capable of degrading l-malic acid.

These new yeasts were respectively designated MB7TCα, MBII, MBK and MBO.

The invention also concerns a process for the reduction of malic acidity in wine making.

A wine is considered acid when it contains an elevated concentration of l-malic acid, which in turn is produced by fermentation of musts from not fully ripe grapes. L-malic acid is one of the acids present in musts and wines, together with tartaric acid, citric acid, and much smaller amounts of oxalic acid, uronic acid, succinic acid, lactic acid, acetic acid, glioxalic acid, gyleric acid, pyruvic acid, oxaloacetic acid, alpha-ketoglutaric acid, etc.

The two major acids in terms of concentration, tartaric acid and malic acids, are metabolized to sugar during the process of grape maturation on the wine. At the end of maturation, malic acid undergoes the greatest rate of degradation, with concentrations being reduced from 200 mEq/l to less than 50 mEq/l, while tartaric acid is degraded from 150-200 mEq/l to 100-150 mEq/l. The metabolization of tartaric acid leads to its lowest concentrations almost one month before the end of grape maturation, and malic acid degradation continues to increase up to full grape maturation. As mentioned earlier, acid musts result from the harvesting of grapes that did not go through the full maturation process. Thus, when fermented, these musts also result in acid wines.

Since tartaric acid present in must is stable to the action of bacteria and yeasts, the correction of acidity in wines by biological processes is limited to malic acid degradation. Microbial degradation of malic acid can occur through the action of bacteria of the genera Pediococcus, Lactobacillus and Leuconostoc via malolactic fermentation, with the formation of lactic acid, or by degradation to ethanol via the action of yeasts that develop maloalcoholic fermentation.

Malolactic fermentation primarily occurs in wines with acidity of less than 1 g/l tartaric acid and pH higher than 3.1. Fermentation with homofermenting bacteria of highest interest for wine should occur at pH between 3.2 and 3.3 (MENDOZA A.A. RODRIGUEZ J.C. (1981), Valor Enologico de la Acidez Total de Valoracion, pH y Estado Fisioquimico de los Acidos en el Vino, In : Iº Simposio de Enologia e Viticultura, Edited par Universidade de Caxias do Sul, Caxias do Sul, Brasil, 106 pages).

Schizosaccharomyces pombe yeasts have been used to decrease malic acid in wine by CASTELLI T. (1969), Il Vino al Microscopio, Luigi Scialpi Editore, Roma, 248 pages ;, CASTELLI T. & HAZNEDARI S. (1971), Considerazioni sulla Fermentazione Maloalcolica, Risultati Ottenuti nel Quadriennio, Atti. accad. Ital. Vite e Vino, 23:177-202 ; BENDA I. & SCHMITT A. (1966), Onologische Untersuchungen zum Biologischen Säureabbau in Most durch Schizosaccharomyces pombe, Weinberg und Keller-Band, 13:239-253 ; BENDA I. (1974), Les Schizosaccharomyces et leur effect desacidifiant en vinification, Vignes et Vins, numéro special au colloque international d'oenologie d'Arc et Senans, 31-36 ; HUGLIN P. MEYER J.P. & SCHAEFFER A (1976), Elaboration de concentrés de mouts de raisins peu acides à l'aide de levures du genre Schizosaccharomyces, C.R.S. l'Acad. d'Agr. de France, 6:412-419 ; GALLANDER J.P. (1977), Deacidification of eastern table wines with Schizosaccharomyces pombe, Am. J. Enol. Viticult. 28:65-68 ; and CARRAU J.L. (1981), Schizosaccharomyces pombe : Condiçoes para sua Utilizaçao a Nivel Industrial, 1º Simpósio de Enologia e Viticultura, edited by Universidade de Caxias do Sul, Caxias do Sul, Brasil, 106 pages. However, owing to problems related to the growth rate of Schizosaccharomyces pombe which require the use of large inocula for wine fermentation (CARRAU J.L. : Azevedo J.L. : Sudery P. e Campbell D., Methods for Recovering Fusion Products Among Oenological Strains of Saccharomyces cerevisiae and Schizasaccharomyces pombe - Rev. Brasil, Genet. 5(1):221-226 - 1982), the use of this yeast for demalification is not practical for common application in wineries. Indeed, one of the objectives of the present invention was to construct yeasts by fusing spheroplasts of Saccharomyces cerevisiae Montrachet (having a greater growth rate) and Schizosaccharomyces pombe Benda I. These yeasts can solve the problems outlined above because their growth rate is more rapid than that of the parental strain Schizosaccharomyces pombe and because they express the ability to degrade 1-malic acid in addition to presenting part of the genome of yeasts traditionally used in wine fermentation.

A decrease in malic acid content also favors the natural development of malolactic fermentation which is known to be a determinant of the biological stabilization of wines.

The present invention was based on the use of microbial genetics, to whose methods protoplast fusion was added during the 1970's. This method permits the exchange of genetic material between cells that are not compatible for crossing. For yeasts, protoplast fusion utilizes lytic enzymes from the digestive juice of Helix aspersa which produce the formation of cells fully (protoplasts) or partially (spheroplasts) devoid of walls. Both protoplasts and spheroplasts can be easily fused in the presence of polyethyleneglycol, as initially observed by VAN SOLINGEN P. and VAN DER PLAAT J.R. (1977), Fusion of Yeast Spheroplasts, Journal of Bacteriology, 130:946-947. The fusion products are then submitted to conditions that will lead to the reestablisment of the cell wall. These conditions include a regenerating medium containing Ca⁺⁺ and a large osmotic concentration, with inoculation onto pour-plates yielding the best percentage of regeneration. This technique has been used previously to obtain fusion products of technological interest (RUSSELL I. & STEWART G.G. (1979), Spheroplast Fusion of Brewer's Yeast Strain, J. Inst. Brew, 85:95-98 ; TUBB R.S. (1979), Applying Yeast Genetics in Brewing, A Current Assessment, J. Inst. Brew, 85:286-289) . Modern wine making outfits use selected yeasts that are applied to the fermentations by the use of starter. CARRAU (1982), ibid, reported the demalifying ability of 6 different strains of Schizosaccharomyces pombe, which degraded 55 to 75 % of malic acid within 24 hours and 83.8 to 98.8 % after 72 hours. On the basis of this ability of Schizosaccharomyces pombe to degrade malic acid, CARRAU (1981), ibid, proposed large-scale fermentations using this yeast to degrade malic acid in wine. This process would utilize inocula of 500 x 10⁵ cells of Schizosaccharomyces pombe in order to obtain parcial malic acid degradation during wine fermentation.

The methodologies that constitute the inventions are reported below :
1. Selection of genotypes to obtain the first fusion product (MB7TCα).
A strain of Schizosaccharomyces pombe : called Benda I was selected as the yeast with the most effective ability to degrade malic acid. As a yeast of enologic use which produces good organoleptic characteristics in wines, we selected the strain Saccharomyces cerevisiae denominated Montrachet.
2. Selection of markers for obtaining MB7TC
The Schizosaccharomyces pombe strain does not grow on galactose as the single source of carbon and its growth on WLN (Difco) gives origin to greenish colonies. The Montrachet strain of Saccharomyces cerevisiae forms white colonies when growing in WLN medium.
3. Production of spheroplasts.
This invention was based on the technique of GOODEY (1980), ibid, except that 0.8 M KCl was substituted for 1 M sorbitol as osmotic buffer.
4. Spheroplast fusion and regeneration.
After the appearance of spheroplast levels higher than 85 % in the two suspensions, the latter were concentrated by centrifugation and the pellets pooled in a single tube and treated with 2 ml of solution of 37 % polyethyleneglycol 4,000 (GOODEY (1980), ibid).
5 . Recovery of fusion products.
After the fusion treatment, the cells were regenerated in regenerating medium (6.67 g YNB, 20 g glucose, 180 g sorbitol, 1.11 g CaCl₂, and distilled water to 1,000 ml).
The colonies formed were observed microscopically to determine the occurrence of colonies with irregular borders, called "hairy" colonies. Colony coloration was also observed in WLN medium and colonies with irregular borders that also showed colors differing from those of the parental strains in WLN were studied for capacity to degrade malic acid.
6. Production of MBO, MBII and MBK.
In order to increase the genotypic contribution of Saccharomyces to the fusion products, strain MB7TC was fused with strains O Mendoza and KI, both of them Saccharomyces cerevisiae, and also with the parental strain Montrachet.
The methods for obtaining new fusion products were the same as those described for MB7TC.
7. Evaluation of demalifying ability.
The clones obtained by spheroplast fusion were studied in test tubes and in 1,000-ml Erlenmayer flasks containing grape must. A racemic mixture of malic acid (10 g/l) was added to the musts for the study of malic acid degradation ability. The results for clone MB7TC with respect to fermentation ability, as indicated by CO₂ release with the use of Muller valves by the method of CASTELLI (1969), ibid, and malic acid degradation are shown in Figure I and Table I, respectively. It can be seen the fermenting rate of MB7TCα is higher than that of the parental Schizosaccharomyces pombe strain, and that its demalifying ability is good.
8. How these fusion products can be used by the wine making industry.
In wine making, the concentrations of native yeast cells after the extraction of grape juice often reach values higher than 10⁶ cells/ml, a fact implying that the yeasts described in the present invention need to be inoculated at similar concentrations for their demalifying ability to be expressed. The present invention proposes a wine making process utilizing inocula 0f 100 x 10⁵ cells/ml fusion products at the beginning of fermentation in order to obtain wines with partial degradation of l-malic acid.

**TABLE I -**

| | Malic acid degradation by the fusion product M B7TCα and the parental strains Montrachet and Benda I,expressed as mEq/l and % total. | | | |
|---|---|---|---|---|
| | 72 hours | | 144 hours | |
| Strains | mEq/l | % | mEq/l | % |
| Montrachet | 0 | 0 | 0 | 0 |
| Benda I | 47 | 70 | 48 | 72 |
| MB7TCα | 25 | 37 | 55 | 81 |

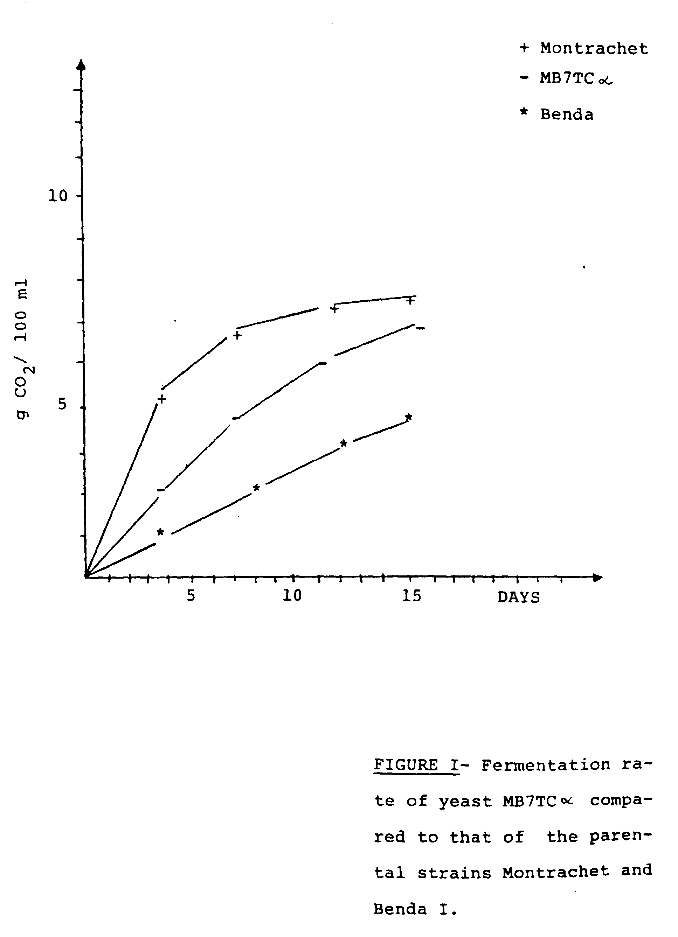

## Claims

1. Yeasts characterized by the fact that they are obtained by fusion between Saccharomyces cerevisiae (Montrachet) and Schizosaccharomyces pombe (Benda I), that they grow more rapidly than the parent strain Schizosaccharomyces pombe, that they present part of the genome of yeasts traditionally used in wine fermentation, and that they have the ability to degrade 1-malic acid.

2. Yeast characterized by the fact that it is obtained by fusion between a yeast according to Claim 1 and the parental strain Saccharomyces cerevisiae (Montrachet), that it grows more rapidly than the parent strain Schizosaccharomyces pombe, that it presents part of the genome of yeasts traditionally used in wine fermentation, and that it has the ability to degrade 1-malic acid.

3. Yeast characterized by the fact that it is obtained by fusion between a yeast according to Claim 1 and a Saccharomyces cerevisiae yeast with killer phenotype, that it grows more rapidly than the parent strain Schizosaccharomyces pombe, that it presents part of the genome of yeasts traditionally used in wine fermentation, and that it has the ability to degrade 1-malic acid.

4. Yeast characterized by the fact that it is obtained by fusion between a yeast according to Claim 1 and a Saccharomyces cerevisiae yeast denominated O Mendoza, that it grows more rapidly than the parent strain Schizosaccharomyces pombe, that it presents part of the genome of yeasts traditionally used in wine fermentation, and that it has the ability to degrade 1-malic acid.

5. Yeast according to any of the preceding Claims characterized by the fact that it is utilized in wine making processes.

6. A process for the reduction of malic acidity in wine making characterized by the fact that it utilizes yeasts obtained according to any of Claims 1 to 4.

7. Process for the reduction of malic acidity according to claim 6 characterized by the fact that it utilizes concentrations of 100 X 10⁵ cells of any of the yeasts according to the preceding Claims, as an inoculum to be added to the must at the beginning of fermentation.

## Patentansprüche

1. Hefen, dadurch gekennzeichnet, dass sie durch Fusion von *Saccharomyces cerevisiae* (Montrachet) und *Schizosaccharomyces pombe* (Benda I) erhalten werden, dass sie schneller als der elterliche Stamm *Schizosaccharomyces pombe* wachsen, dass sie einen Teil des Genoms der herkömmlicherweise bei der Weingärung verwendeten Hefen aufweisen und dass sie die Fähigkeit besitzen, 1-Apfelsäure abzubauen.

2. Hefe, dadurch gekennzeichnet, dass sie durch Fusion einer Hefe nach Anspruch 1 und des elterlichen Stamms *Saccharomyces cerevisiae* (Montrachat) erhalten wird, dass sie schneller als der elterliche Stamm *Schizosaccharomyces pombe* wächst, dass sie einen Teil des Genoms der herkömmlicherweise bei der Weingärung verwendeten Hefen aufweist und dass sie die Fähigkeit besitzt, 1-Apfelsäure abzubauen.

3. Hefe, dadurch gekennzeichnet, dass sie durch Fusion einer Hefe nach Anspruch 1 und einer *Saccharomyces cerevisiae*-Hefe mit Killer-Phänotyp erhalten wird, dass sie schneller als der elterliche Stamm *Schizosaccharomyces pombe* wächst, dass sie einen Teil des Genoms der herkömmlicherweise bei der Weingärung verwendeten Hefen aufweist und dass sie die Fähigkeit besitzt, 1-Apfelsäure abzubauen.

4. Hefe, dadurch gekennzeichnet, dass sie durch Fusion einer Hefe nach Anspruch 1 und einer O Mendoza genannten *Saccharomyces cerevisiae-Hefe* erhalten wird, dass sie schneller als der elterliche Stamm *Schizosaccharomyces pombe* wächst, dass sie einen Teil des Genoms der herkömmlicherweise bei der Weingärung verwendeten Hefen aufweist und dass sie die Fähigkeit besitzt, 1-Apfelsäure abzubauen.

5. Hefe gemäss einem beliebigen der vorangehenden Ansprüche, dadurch gekennzeichnet, dass sie in Weinherstellungsverfahren verwendet wird.

6. Verfahren zur Verminderung der Apfelsäure-Azidität in der Weinhersteilung, dadurch gekennzeichnet, dass es Hefen verwendet, die gemäss einem beliebigen der Ansprüche 1 bis 4 erhalten wurden.

7. Verfahren zur Verminderung der Apfelsäure-Azidität gemäss Anspruch 6, dadurch gekennzeichnet, dass es Konzentrationen von 100 × 10⁴⁵ Zellen einer beliebigen Hefe nach den vorangehenden Ansprüchen als ein dem Most zu Beginn der Gärung hinzuzufügendes Inokulum verwendet.

## Revendications

1. Levures caractérisées en ce qu'elles sont obtenues par fusion entre Saccharomyces cerevisiae (Montrachet) et Schizosaccharomyces pombe (Benda I), et qu'elles croissent plus rapidement que la souche parente Schizosaccharomyces pombe, qu'elles présentent une partie du génome des levures traditionnellement utilisées dans la fermentation du vin, et qu'elles ont la capacité de dégrader l'acide 1-malique.

2. Levure caractérisée en ce qu'elle est obtenue par fusion entre une levure selon la revendication 1 et la souche parente Saccharomyces cerevisiae (Montrachet), qu'elle croît plus rapidement que la souche parente Schizosaccharomyces pombe, qu'elle présente une partie du génome des levures traditionnellement utilisées dans la fermentation du vin, et qu'elle a la capacité de dégrader l'acide 1-malique.

3. Levure caractérisée en ce qu'elle est obtenue par fusion entre une levure selon la revendication 1 et une levure Saccharomyces cerevisiae ayant un phénotype tueur, qu'elle croît plus rapidement que la souche parente Schizosaccharomyces pombe, qu'elle présente une partie du génome des levures traditionnellement utilisées dans la fermentation du vin, et qu'elle a la capacité de dégrader l'acide 1-malique.

4. Levure caractérisée en ce qu'elle est obtenue par fusion entre une levure selon la revendication 1 et une levure Saccharomyces cerevisiae dénommée O Mendoza, qu'elle croît plus rapidement que la souche parente Schizosaccharomyces pombe, qu'elle présente une partie du génome des levures traditionnellement utilisées dans la fermentation du vin, et qu'elle a la capacité de dégrader l'acide 1-malique.

5. Levure selon l'une des revendications précédentes, caractérisée par le fait qu'elle est utilisée dans les procédés de fabrication du vin.

6. Procédé pour la réduction de l'acidité malique dans la fabrication du vin, caractérisé par le fait qu'il utilise des levures obtenues selon l'une des revendications 1 à 4.

7. Procédé pour la réduction de l'acidité malique selon la revendication 6, caractérisé en ce qu'il utilise des concentrations de 100 X 10⁵ cellules de l'une quelconque des levures selon les revendications précédentes, en tant qu'inoculum à ajouter au moût en début de la fermentation.
